# EUROPEAN PATENT APPLICATION

(11) **EP 1 666 050 A1**
(43) Date of publication of application: **07.06.2006**
(21) Application number: 03818319.0
(22) Date of filing: 26.08.2003
(51) Int. Cl.: A61K 35/74, A61P 1/16

(54) **USE OF ENREROCOCCUS FAECIUM STRAINS FOR CURING HEPATIC INSUFFICIENCY AND FOR REGENERATING AND INTENSIFYING METABOLISM IN A LIVER**

(71) Applicant: Obschestvo S Ogranichennoi Otvetstvennostyu Alef- Farma, Moscow, 123557 (RU)
(72) Inventor: BAKULIN, Igor Gennadievich, Moscow, 123368 (RU); NOVOZHENOV, Vladislav Grigorievich, Moscow, 113623 (RU); PARFENOV, Alexei Nikolaevich, Moskovskaya obl., 141300 (RU); USHAKOV, Igor Borisovich, Moscow, 105077 (RU); FEDIN, Anton Viktorovich, Moscow, 127410 (RU)
(74) Representative: HOFFMANN EITLE
(86) International application number: PCT/RU2003/000386
(87) International publication number: WO 2005/018654

(57) **Abstract**

The invention relates to the use of enterococcus faecium (VKPM B-3490) and enterococcus faecium M-3185 (VKPM B-349i) strains, a composition and a Bilaktin preparation based on said stains for curing a hepatic insufficiency which is exhibited by hepatodepressive, cytolytic and cholestatic syndromes and by intrahepatic portal hypertension during hepatic diseases and liver injury, for liver regeneration, preventing overstress in extreme environment conditions, for improving the aerobic productivity of an organism, for increasing a lipid participation in energy supply and for improving correlation between a muscle and fatty mass. Said invention also relates to a method for treating and preventing a hepatic insufficiency exhibited by hepatodepressive, cytolytic and cholestatic syndromes and by intrahepatic portal hypertension during hepatic diseases and liver injury and to a method for improving the aerobic productivity of an organism, for increasing a lipid participation in energy supply and for improving the relation between a muscle and fatty mass.

## Description

### Field of the Invention

The present invention relates to medicine and biotechnology, and more particularly to use of *Enterococcus faecium* M strain (VKPM B-3490) and *Enterococcus faecium* M-3185 strain (VKPM-3491) (All-Union Collection of Industrial Microorganisms, currently - Russian National Collection of Industrial Microorganisms) and of a composition based thereupon for treating (curing) hepatic deficiency comprising hepatodepressive, cytolytic and cholestatic syndromes, intrahepatic portal hypertension developing in case of liver diseases and injuries induced by viral, toxic and other mechanisms of development, for liver regeneration, as well as for preventing overstrain syndrome under extreme environmental conditions and for intensifying metabolic processes in the liver.

### Background of the Invention

Methods for treating hepatic deficiency, said methods comprising use of hepatoprotective agents, glucocorticosteroid preparations and/or immunomodulating means are known in the art (Nikitin I.G., Storozhakov G. I.//Clinical Prospects in Gastroenterology, Hepatology. - 2001, No 3, pp. 7-11; Katikova O.Yu.et al. //Problems of Medical Chemistry.- 2001, vol. 47, Issue 6; Robert P Perrillo et al.// Hepatology.-1999, vol. 30.- No. 4, Pt. 2: 301A (Abstract No. 561); Glue P. et al.//Clinical Pharmacological Therapy. - 2000, vol. 68, pp. 556-567). Said methods are disadvantageous by their limited usage both due to the presence of contraindications, especially in sustained administration, and due to low efficiency in other cases.

A method for intensifying metabolic processes in liver and preventing overstrain syndrome under extreme environmental conditions said method comprising using anabolic hormones, antihypoxants and/or actoprotective agents is also known (Bulanov Yu.B., Anabolics. Moscow, 1993). Said methods are disadvantageous by considerable limitation of their use in case of some diseases, particularly hepatic diseases, ranking with the group of doping preparations, origination of side effects in sustained administration.

Use of *Enterococcus faecium* M strain (VKPM B-3490) and *Enterococcus faecium* M-3185 strain (VKPM-3491) as well as a composition based thereupon comprising said strains in a ratio of 0.5 to 1.5 and 1.5 to 0.5 as an immunostimulating agent increasing immune status, viral disease resistance, stress resistance is known in the art (PCT/RU96.00132).

The present invention is directed to overcoming said disadvantages of the known methods for treating hepatic deficiency, intensifying metabolic processes in liver and preventing overstrain syndrome under extreme environmental conditions by using as medicinal and prophylactic agents in case of liver diseases and injuries, as well as for intensifying metabolic processes in liver and for prophylaxis of overstrain syndrome, by administering *Enterococcus faecium* M strain (VKPM B-3490) and *Enterococcus faecium* M-3185 strain (VKPM B-3491), a composition and the "Bilaktin" preparation based thereupon.

### Summary of the Invention

The authors have unexpectedly discovered high therapeutic efficiency of previously known *Enterococcus faecium* M strain (VKPM B-3490) and *Enterococcus faecium* M-3185 strain (VKPM B-3491) and previously known composition for treatment and prophylaxis of liver diseases and injuries, for prophylaxis of overstrain and for intensification of metabolic processes in liver.

The present invention provides use of *Enterococcus faecium* M strain (VKPM B-3490) and *Enterococcus faecium* M-3185 strain (VKPM B-3491), the composition and the "Bilaktin" preparation based thereupon, for treating hepatic deficiency comprising hepatodepressive, cytolytic and cholestatic syndromes, intrahepatic portal hypertension developing in case of liver diseases and injuries, for liver regeneration, for preventing overstrain syndrome under extreme environmental conditions; for improving aerobic productivity of organism and for increasing the degree of involvement of lipids in energy supply and improving the ratio of body muscle weight to body fat weight.

The invention further provides a method for treating and preventing hepatic deficiency comprising hepatodepressive, cytolytic and/or cholestatic syndromes and intrahepatic portal hypertension in liver diseases and injuries; a method for improving aerobic productivity of organism and a method for increasing the degree of involvement of lipids in energy supply and improving the ratio of body muscle weight to body fat weight.

### Detailed Description of the Invention

The liver, being the largest gland in an human body, participates in the processes of digestion, metabolism, blood circulation, performing specific protective, detoxication, enzymatic and elimination functions directed to maintaining constancy of the internal environment. Infections of different etiology, effects of toxic substances, mechanical injuries, extreme environmental factors (including considerable physical loads, elevated temperature), to mention only few, are responsible for the origination of functional disorders, liver diseases and injuries. Dysfunctions of the liver are caused to a considerable extent by changes in the permeability of the cellular membranes of hepatocytes, which lead to derangement of hepatic metabolism and to an increased content of certain substances in blood, monitoring the content thereof being used in diagnosing liver diseases and injuries. Such substances include:
- alanine-aminotransferase (ALT),
- aspartate-aminotransferase (AST),
- gamma-glutamyltranspeptidase (GGTP),
- alkali phosphatase (AP),
- bilirubin,
- cholesterol.

The liver is responsible for crucial steps in carbohydrate, protein and fat metabolisms. The level of aerobic productivity of an organism is directly connected with the rate of conversion of lactate to glycogen which takes place in the liver in the Cori cycle: it is known that during intensive work in muscles due to insufficient oxygen supply to provide energy consumption and ADP-to-ATP reduction there takes place a process of anaerobic digestion of carbohydrates (without O₂ participation) - glycolysis. The resultant lactic acid incomes into the blood to be converted to glycogen upon entering blood circulation into the liver. About 20% of the lactic acid in the liver, in the presence of oxygen, is oxidized to carbon dioxide and water, thereby providing energy for the process of lactic acid conversion to glycogen. When the physical load level exceeds the value, called the aerobic threshold, at which the functional possibilities of the liver provide the conversion of all the resultant lactate to glycogen, lactate starts accumulating in the organism, the pH of the blood is reduced, metabolic processes are disturbed, energy reserves become rapidly depleted and, as a result, muscle fatigue sets in, down to complete loss of the ability to accomplish physical load.

The liver ensures oxidation of at least 60% of the total amount of fatty acids for providing the energy needs of an organism. Large amounts of intermediate products are formed during oxidation of fatty acids such as acetone and b-hydroxybutyric acid, acetoacetic acid, acetooxybutyric acid ("ketone bodies") which are called "fatigue toxins". Further dergadation of ketone bodies preliminarily requires considerable energy consumption. With intensive physical loads, under the conditions of glucose shortage, the latter being required at least in small amounts for "burning" the ketone bodies, the process of oxidation of fatty acids is blocked at the stage of formation of ketone bodies which, along with lactic acid, shift the pH of the blood to the acid side and form a fatigue syndrome. Under the conditions when the carbohydrate and fat energy sources of an organism are exhausted or cannot make up the required energy expenditures, the organism begins using protein substances for the purpose, a factor that eventually results in emaciation of the muscular system.

Under normal conditions mitosis of hepatic cells (hepatocytes) occurs rather rarely cells (under normal conditions hepatocyte mitoses rate is 0.0003%). Stimulation of mitotic activity of hepatocytes ensures of the completeness of mitotic processes contributes to timely renewal of the hepatic cells, to replacement of damaged cells, to restoration of lobules and segments.

The use of the known *Enterococcus faecium* M strain (VKPM B-3490) and *Enterococcus faecium* M-3185 strain (VKPM B-3491) and of the composition based thereupon for treatment and prophylaxis of different diseases and functional disorders of liver has *unexpectedly* made it possible to shorten, with a high therapeutic effect, the periods of treatment of hepatic deficiency, to enhance the activity of regenerative processes in the liver, to decrease the degree of progression of hepatic dysfunctions; to correct immune system disorders, to improve aerobic productivity of organism, to increase the degree of involvement of lipids in energy supply and to improve the ratio of body muscle weight to body fat weight.

The present invention provides the therapeutic regimen for treating hepatic deficiency and for liver regeneration according to the prototype additionally or in the form of monotherapy, as well as for increasing aerobic productivity of an organism, increasing the degree of involvement of lipids in energy supply, improving the ratio of body muscle weight to body fat weight and preventing overstrain syndrome, a preparation is prescribed to patients for oral administration, said preparation containing concentrates of monocultures of viable *Enterococcus faecium* M strain (VKPM B-3490) and *Enterococcus faecium* M-3185 strain (VKPM B-3491) in a dose of at least 10¹¹ CFU, one or three times a day during 10 to 90 days or an entire period the extreme factors are in effect, followed by required rehabilitation period.

The use of said strains and composition based thereupon for treating hepatic deficiency makes it possible to reduce considerably the duration of hepatodepressive, cytolytic and cholestatic syndromes, to improve the synthetic function of liver, to correct immunity disorders, to stimulate energy processes, this contributing to an appreciable improvement in the results of treatment and the quality of patients' life.

The use of said strains and composition based thereupon for liver regeneration makes it possible to stimulate the mitotic activity of hepatocytes, to ensure completeness of mitotic process and to increase the rate of liver regeneration.

The use of said strains and composition based thereupon for the prophylaxis of overstrain syndrome, for improving aerobic productivity of an organism, for increasing the degree of involvement of lipids in energy supply and improving the ratio of body muscle weight to body fat weight makes it possible to increase the lactate-to-glucose reduction rate in the liver in the Cori cycle, to intensify the cell metabolism of hepatocytes with an increase of the proportion of lipids participating in energy supply.

Taking into account the high efficiency of *Enterococcus faecium* M strain (VKPM B-3490) and *Enterococcus faecium* M-3185 strain (VKPM B-3491) in treating hepatic deficiency and accelerating liver regeneration, improving aerobic productivity of an organism, increasing the degree of involvement of lipids in energy supply, improving the ratio of body muscle weight to body fat weight and preventing overstrain syndrome, "Bilaktin" preparation (Registration Certificate No 004339.P.643.07.2002) was developed on the basis of these strains.

The "Bilaktin" preparation usually is manufactured in a form of capsules and/or tablets. Said preparation comprises:
lyophilically dried bacterial mass of *Enterococcus faecium* M (VKPM B-3490), 40-60%
lyophilically dried bacterial mass of *Enterococcus faecium* M-3185 (VKPM B-3491), 40-60%
a residual amount of soluble dry substances of culture medium, up to 20%

The invention is illustrated by preferable examples which are intended only to suppor the invention and cannot be considered as a basis for limiting the scope of the Applicant's claims. A person skilled in the art will easily find possibilities for other embodiments of the invention which no, doubt, come within the scope of the Applicant's claims as presented hereinbelow..

### Example 1

Patient G., female, 38 years old, underwent hospital treatment in a gastroenterological department from 11.12.2002 through 27.12.2002.

Diagnosis: primary biliary cirrhosis, class A-B, moderate activity. Hepatolienal syndrome. Hepatic deficiency 1-2 deg. Hepatic encephalopathy 0.

Complaints: feeling of asthenia in the evenings, pronounced skin itch, dyspeptic disorders, yellowing of skin integuments.

From the anamnesis: Considers herself to be sick during 4-5 years. For the last few years was repeatedly hospitalized with the diagnosis: "Hepatic cirrhosis, cryptogenic, moderate activity".

Treatment: Essentiale, Karsil, Legalon, in courses without positive dynamics of manifestations of cytolytic and cholestatic syndromes.

Clinical treatment: infusion therapy, enzymatic therapy, vitamins. Since the moment of admission of the patient, the "Bilaktin" preparation was prescribed, which contained concentrates of *Enterococcus faecium* M (VKPM B-3490) and *Enterococcus faecium* M-3185 (VKPM B-3491) monocultures, 2 capsules, 3 times a day. Administration of the "Bilaktin" preparation was continued after the patient was discharged from the hospital: 2 capsules, 3 times a day, up to one month.

With the treatment carried out as described above, the skin itch disappeared after one week, the overall health (sleep, workability) improved, manifestations of dyspeptic disorders abated. After one month of intaking the preparation, ALT and AST indices and all indices of the GAM scale (General physical and mental state/Activity/Mood scale) improved. Hepatic sonography results showed a decrease in the size of the liver.

**Biopsy of the liver: primary biliary cirrhosis, Knodell score 8**

| | ALT | AST | Bilirubin | AP | GGTP |
|---|---|---|---|---|---|
| Before treatment | 118 | 116 | 33 | 1693 | 751 |
| After 1 week | 77 | 119 | 21 | 2174 | |
| After 1 month | 80 | 11 | 14 | 1770 | 548 |

The patient was discharged from the hospital with significant improvement.

### Example 2

Patient M., female, 38 years old, underwent treatment in a gastroenterological department from 17.10.2002 through 14.11.2002.

Diagnosis: hepatic cirrhosis, exotoxic etiology, class B-C, pronounced activity.

Complaints: feeling of asthenia in the evenings, skin itch, duapeptic disorders, increased size of the abdomen.

From the anamnesis: considers herself to be sick during 2-3 years.

Hospitalized with the diagnosis: subcompensated cirrhosis of the liver.

Course of treatment: Heptral, Duphalac, infusion therapy. After one week of such treatment, deterioration of laboratory indices was observed, and therefore "Bilaktin" was prescribed to the patient: 2 capsules 3 times a day, till completion of the hospital treatment. Heptral and Duphalac were withheld.

7 days after the administration of the "Bilaktin" preparation in the form of monotherapy, the skin itch disappeared, the state of health improved (sleep, physical tonus, workability), manifestations of dyspeptic disorders abated.

After one month, the laboratory indices characterizing cytolytic and cholestatic syndromes became normal, all the indices according to the GAM scale improved.

**Laboratory investigations data:**

| | ALT | AST | Bilirubin | AP | GGTP |
|---|---|---|---|---|---|
| Before treatment | 330 | 375 | 94 | 490 | 405 |
| After 1 week | 345 | 395 | 67 | 290 | 255 |
| After 2 weeks | 74 | 82 | 52 | | 106 |
| After 1 month | 7 | 43 | 24 | 200 | 17 |

The patient was discharged from the hospital in good condition; the "Bilaktin" preparation was recommended for use in a dose of 1 capsule a day during one month.

### Example 3

Patent M., male, 27 years old.

Diagnosis: non-alcoholic steatohepatitis, moderate activity.

Complaints: non-motivated feeling of asthenia in the day-time and in the evening.

From the anamnesis: In 2001, during biochemical examination of the patient blood, anti HBs Ab were detected in patient blood while the blood was negative for HBV-DNA. The biopsy sample of the liver hepatic tissue was positive for HBV-DNA. The patent received a course of antiviral treatment: Lamivudine 100 mg/day + Rheoferon, 6 mln AU x 3 times a week (for 6 months); 6 months later no HBV-DNA was found in the biopsy sample of the liver. Despite the treatment, cytolytic syndrome remained in the patient: ALT, 140; AST, 80; bilirubin, 25; this being the reason for the out-patient administration of the "Bilaktin" preparation (2 capsules, 3 times a day during one month, followed by changeover to 1 capsule, once a day during one month).

With the "Bilaktin" therapy, the state of health improved after one week (sleep, workability, attention); good workability remained well preserved after one month.

Biopsy of the liver: the aspect of chronic hepatitis, fatty degeneration to 5%, Knodell score 9.

**Laboratory investigations data:**

| | ALT | AST | Bilirubin | AP | GGTP |
|---|---|---|---|---|---|
| Before treatment | 140 | 80 | 25 | 180 | 58 |
| After 1 week | 98 | 65 | 21 | 120 | 45 |
| After 2 weeks | 90 | 45 | 12 | 91 | 35 |
| After 1 month | 23 | 36 | 18 | 150 | 40 |

### Example 4

The "Bilaktin" preparation was administered to nondescript male albino rats and to nondescript male white mice, intragastrically for 7 days, in doses D1=10 g/kg, D2=30 g/kg, D3=70 g/kg. After sacrificing the animals by decapitation, morphological investigations were carried out. Examination of macropreparations revealed no changes in the organ. Microscopic examination revealed hepatic cells in the phase of numerous mitoses in different phases from the prophase and metaphase to the telophase, this being not characteristic of normally amitotic hepatic cells (under normal conditions hepatocyte mitoses rate is 0.0003%). Numerous binuclear hepatocytes were also revealed, this being indicative of pronounced stimulation of mitotic activity of hepatocytes, completion of the mitosis and liver regeneration.

### Example 5

Within the framework of a training camp course, against a background of high standard loads, five highly qualified sportsmen received the "Bilaktin" preparation (main group) during three weeks and other five highly qualified sportsmen received a placebo (control group). There were carried out: a clinical-laboratory and physical examination, skinfold caliper measurements during a 6-minute run on a treadbahn with determining the number of heart contractions, measuring arterial pressure; general workability, lactic acid glucose content, some biochemical indices characterizing the hepatic function were determined, the indices characterizing the muscle and fat mass of the body under the conditions of high temperature and low air humidity in the beginning and the end of a 3-week training cycle.

From the results of final testing in the main group there were found a higher level of workability (Cooper's test), absence of an increase in lactate as the volume of loads increases, stabilization of the muscle weight with the fat mass reduced.

The level of biochemical indices including those characterizing the function of the liver (AST, ALT, urea) varied only within the normal range, while in the sportsmen of the control group an increase in the ALT level was registered above the norm in two cases and an increase in the AST level in three cases. The cholesterol level in the sportsmen of the experimental group after a 3-week training course remained within the norm, while in two sportsmen of the control group said index exceeded the normal value on completion of the training camp course.

| Indices | | Groups | | | |
|---|---|---|---|---|---|
| | | Main | | Control | |
| | | Before administration | After administration | Before administration | After administration |
| Distance length | m | 2105±9.8 | 2209±13.2 | 2114±12.2 | 2138+10.6 |
| | increase, % | +4.9 | | +1.1 | |
| Lactate level | mMole/l | 8.96±0.2 | 9.08±0.2 | 8.78±0.12 | 9.81±0.16 |
| | changes,% | +1.33 | | +11.73 | |
| Body weight | kg | 65.8±0.4 | 64.6±0.2 | 66.1±0.25 | 64.,8±0,4 |
| | changes, % | -1.82 | | -1.96 | |
| Muscle mass | kg | 34.15±0.16 | 33.89±0.1 | 34.23±0.12 | 32.48±0.2 |
| | changes, % | -0.76 | | -5.11 | |
| Fat mass | kg | 5.85±0.12 | 5.29±0.24 | 5.9±0.2 | 5.57±0.2 |
| | changes, % | -9.57 | | -5.59 | |

### Example 6

"Bilaktin" preparation was developed on the basis of a composition of *Enterococcus faecium* M strain (VKPM B-3490) and *Enterococcus faecium* M-3185 strain (VKPM B-3491). This preparation is used for treating hepatic deficiency and for liver regeneration, improving aerobic productivity of an organism, increasing the degree of involvement of lipids in energy supply, improving the ratio of body muscle weight to body fat weight and preventing overstrain syndrome. A distinctive feature of the preparation is the high concentration of viable cells (colony forming units - CFU/g) which is achieved by using special media and cultivation conditions, as well as methods of concentration and drying, which allow preserving the high viability of bacterial cells without applying special protective media. The preparation has passed a comprehensive complex of investigations for harmlessness and was assigned to the fourth class of toxicity (non-toxic substances) and to the third class of allergization (weak allergens). The preparation is registered with the Ministry of Public Health of the Russian Federation as a biologically active food additive (BAA) - Registration Certificate No 004339.P.643. 07.2002.

The preparation is manufactured in the form of capsules containing dry powder-like mass of viable bacterial cells of *Enterococcus faecium* M strain (VKPM B-3490) and *Enterococcus faecium* M-3185 strain (VKPM B-3491) packed in flasks or in the form of tablets prepared by drying frozen shaped paste of monocultures of *Enterococcus faecium* M strain (VKPM B-3490) and *Enterococcus faecium* M-3185 strain (VKPM B-3491). The preparation is administered perorally.

### Industrial applicability

*Enterococcus faecium* M strain (VKPM B-3490) and *Enterococcus faecium* M-3185 strain (VKPM B-3491), the composition of *Enterococcus* faecium M strain (VKPM B-3490) and *Enterococcus faecium* M-3185 strain (VKPM B-3491) and the "BILAKTIN" preparation and the developed methods for treating and preventing diseases and injuries can be used for treating hepatic deficiency (hepatodepressive, cytolytic and cholestatic syndromes) in case of liver diseases and injuries, for liver regeneration, overstrain prevention under extreme environmental conditions, for improving aerobic productivity of an organism, for increasing the degree of involvement of lipids in energy supply of an organism and improving the ratio of body muscle weight to body fat weight.

### REFERENCES

1. Bulanov Yu.B. Anabolics. Moscow, 1993 (in Russian).
2. Ivashkin V.T.//Rossijskij Zhumal Gastroenterologii, Gepatologii, Koloproktologii, 1998, No. 5.
3. Katikova O.Yu. et al. //Voprosy Meditsinskoj Khimii, 2001, vol. 47, issue 6.
4. Nikitin I.G., Storozhakov G.I.//Klinicheskie Perspectivy v Gastroenterologii, Gepatologii, 2001, No. 3, pp. 7-11.
5. Khazanov A.I. Diseases of the Liver, Gall Bladder and Bile Ducts. In: Academician F.I. Komarov (Ed.), Diagnostics and Treatment of Internal Diseases. - Manual for physicians in 3 volumes, "Meditsina" Publishers, 1996, vol. 3, pp. 195-300 (in Russian).
6. Glue P. et al.//Clinical Pharmacological Therapy, 2000, vol. 68, pp. 556-567.
7. Robert P Perrillo et al. //Hepatology, 1999, vol. 30, No 4, Pt 2: 301 A (Abstract No 561).
8. PCT/RU 96/00132 of May 27, 1996 "Use of STREPTOCOCCUS FAECIUM STRAINS AND COMPOSITION CONTAINING THE SAME".

## Claims

1. A use of *Enterococcus faecium* M strain (VKPM B-3490) for treating hepatic deficiency comprising hepatodepressive, cytolytic and cholestatic syndromes, intrahepatic portal hypertension developing in case of liver diseases and injuries.

2. A use of *Enterococcus faecium* M strain (VKPM B-3491) for treating hepatic deficiency comprising hepatodepressive, cytolytic and cholestatic syndromes, intrahepatic portal hypertension developing in case of liver diseases and injuries.

3. A use of *Enterococcus faecium* M strain (VKPM B-3490) for liver regeneration.

4. A use of *Enterococcus faecium* M-3185 strain (VKPM B-3491) for liver regeneration.

5. A use of *Enterococcus faecium* M strain (VKPM B-3490) for preventing overstrain under extreme environmental conditions.

6. A use of *Enterococcus faecium* M-3185 strain (VKPM B-3491) for preventing overstrain under extreme environmental conditions.

7. A use of *Enterococcus faecium* M strain (VKPM B-3490) for improving aerobic productivity of an organism.

8. A use of *Enterococcus faecium* M-3185 strain (VKPM B-3491) for improving aerobic productivity of an organism.

9. A use of *Enterococcus faecium* M strain (VKPM B-3490) for increasing the degree of involvement of lipids in energy supply and for improving the ratio of body muscle weight to body fat weight.

10. A use of *Enterococcus faecium* M-3185 strain (VKPM B-3491) for increasing the degree of involvement of lipids in energy supply and for improving the ratio of body muscle weight to body fat weight.

11. A use of a composition based on *Enterococcus faecium* M strain (VKPM B-3490) and *Enterococcus faecium* M-3185 strain (VKPM B-3491) for treating hepatic deficiency comprising hepatodepressive, cytolytic and cholestatic syndromes, intrahepatic portal hypertension developing in case of liver diseases and injuries.

12. A use of a composition based on *Enterococcus faecium* M strain (VKPM B-3490) and *Enterococcus faecium* M-3185 strain (VKPM B-3491) for liver regeneration.

13. A use of a composition based on *Enterococcus faecium* M strain (VKPM B-3490) and *Enterococcus faecium* M-3185 strain (VKPM B-3491) for preventing overstrain under extreme environmental conditions.

14. A use of a composition based on *Enterococcus faecium* M strain (VKPM B-3490) and *Enterococcus faecium* M-3185 strain (VKPM B-3491) for improving aerobic productivity of an organism.

15. A use of a composition based on *Enterococcus faecium* M strain (VKPM B-3490) and *Enterococcus faecium* M-3185 strain (VKPM B-3491) for increasing the degree of involvement of lipids in energy supply and for improving the ratio of body muscle weight to body fat weight.

16. A use of "Bilaktin" preparation comprising bacterial mass of *Enterococcus* faecium M (VKPM B-3490) in an amount of 40 to 60%, bacterial mass of *Enterococcus faecium* M-3185 (VKPM B-3491) in an amount of 40 to 60% and up to 20% of soluble substances of culture medium for treating hepatic deficiency comprising hepatodepressive, cytolytic and cholestatic syndromes, intrahepatic portal hypertension developing in case of liver diseases and injuries.

17. A use of "Bilaktin" preparation comprising bacterial mass of *Enterococcus* faecium M (VKPM B-3490) in an amount of 40 to 60%, bacterial mass of *Enterococcus faecium* M-3185 (VKPM B-3491) in an amount of 40 to 60% and up to 20% of soluble substances of culture medium for liver regeneration.

18. A use of "Bilaktin" preparation comprising bacterial mass of *Enterococcus* faecium M (VKPM B-3490) in an amount of 40 to 60%, bacterial mass of *Enterococcus faecium* M-3185 (VKPM B-3491) in an amount of 40 to 60% and up to 20% of soluble substances of culture medium for preventing overstrain under extreme environmental conditions.

19. A use of "Bilaktin" preparation comprising bacterial mass of *Enterococcus* faecium M (VKPM B-3490) in an amount of 40 to 60%, bacterial mass of *Enterococcus faecium* M-3185 (VKPM B-3491) in an amount of 40 to 60% and to 20% of soluble substances of culture medium for improving aerobic productivity of an organism.

20. A use of "Bilaktin" preparation comprising bacterial mass of *Enterococcus* faecium M (VKPM B-3490) in an amount of 40 to 60%, bacterial mass of *Enterococcus faecium* M-3185 (VKPM B-3491) in an amount of 40 to 60% and up to 20% of soluble substances of culture medium for increasing the degree of involvement of lipids in energy supply and for improving the ratio of body muscle weight to body fat weight.

21. A method for treating and preventing hepatic deficiency comprising hepatodepressive, cytolytic and/or cholestatic syndromes and intrahepatic portal hypertension developing in case of liver diseases and injuries, said method comprising peroral administration of a composition or preparation based on *Enterococcus faecium* M strain (VKPM B-3490) and *Enterococcus faecium* M-3185 strain (VKPM B-3491) in a single dose of at least 10¹¹ *CFU* one to three times a day during 10 to 90 days.

22. A method for improving aerobic productivity of an organism, said method comprising peroral administration of a composition or preparation based on *Enterococcus faecium* M strain (VKPM B-3490) and *Enterococcus faecium* M-3185 strain (VKPM B-3491) in a single dose of at least 10¹¹ *CFU* one to three times a day during 10 to 30 days or over an entire period the extreme factors are in effect, followed by required rehabilitation period.

23. A method for increasing the degree of involvement of lipids in energy supply and improving the ratio of body muscle weight to body fat weight, said method comprising a peroral administration of a composition or preparation based on *Enterococcus faecium* M strain (VKPM B-3490) and *Enterococcus faecium* M-3185 strain (VKPM B-3491) in a single dose of at least 10¹¹ CFU one to three times a day during sports training sessions or when taking special physical exercises.
